(19) Europäisches Patentamt
European Patent Office
Office européen des brevets

(11) **EP 4 113 147 A1**

(12) **EUROPEAN PATENT APPLICATION**

(43) Date of publication:
**04.01.2023 Bulletin 2023/01**

(21) Application number: **21183368.6**

(22) Date of filing: **02.07.2021**

(51) International Patent Classification (IPC):
**G01R 33/48** (2006.01)   **G01R 33/565** (2006.01)
**A61N 5/10** (2006.01)

(52) Cooperative Patent Classification (CPC):
**G01R 33/56572; A61N 5/1049; G01R 33/4808;**
A61N 5/1067; A61N 2005/1055

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **VESANEN, Panu Tapani**
**5656 AE Eindhoven (NL)**

(74) Representative: **Philips Intellectual Property &
Standards
High Tech Campus 52
5656 AG Eindhoven (NL)**

(54) **TRANSFER OF ORGAN CONTOURS BETWEEN GEOMETRICALLY ACCURATE AND DISTORTED COORDINATE SYSTEMS**

(57) The present disclosure relates to a method for controlling a radiation therapy apparatus intended for radiation therapy of a target volume in a subject. The method comprises: receiving a two-dimensional, 2D, magnetic resonance, MR, image of the target volume for a slice selected according to a slice selecting gradient field. A 3D contour drawn on a 3D MR image of the target volume may be received. The 3D contour has first coordinates. The through plane first coordinates of the 3D contour may be distorted in accordance with a spherical harmonic expansion of the slice selecting gradient field, resulting in a distorted 3D contour having second coordinates. The in-plane coordinates of the selected slice may be determined by an interpolation of second coordinates for obtaining the contour on the 2D MR image. The obtained contour and the 2D MR image may be used for determining a possible movement of the target volume.

Fig. 3

EP 4 113 147 A1

**Description**

FIELD OF THE INVENTION

The invention relates to scanning imaging systems, in particular to a medical analysis system for controlling a radiation therapy by a radiation therapy apparatus of a target volume in a subject.

BACKGROUND OF THE INVENTION

A combined magnetic resonance scanner and linear accelerator (MR-Linac) system allows volumetric imaging of the patient during radiotherapy treatment of cancer. This permits real-time monitoring of a target volume (e.g. a tumor) and patient or organ's movements thereby enabling the tracking of internal organs and target volume(s). Radiation treatment planning; however, is conventionally not performed in real-time during the treatment, but beforehand based on static or dynamic image data, e.g., one or multiple sets of three-dimensional (3D) data. This approach assumes that pre-treatment data of a patient represents the intra-treatment situation. With respect to patient movement, this approach also assumes that the motion behavior stays constant. But it has been observed that different patients show different motion attitudes which change depending on that patient's state e.g., respiration, metabolism, anxiety, tiredness. Andrew Janke, Huawei Zhao, Gary J. Cowin Graham J. Galloway, David M. Doddrell,, Magnetic Resonance in Medicine 52:115-122 (2004), discloses a geometry correction technique using spherical harmonic deconvolution methods to compensate for nonlinear gradient effects on MR images.

SUMMARY OF THE INVENTION

Various embodiments provide for a method for controlling a radiation therapy by a radiation therapy apparatus, medical analysis system, and computer program product, as described by the subject matter of the independent claims. Advantageous embodiments are described in the dependent claims.

According to embodiments, contours defined in the geometrically correct 3D MRI data sets are transferred into the slices of the multi-slice MRI datasets acquired during RT delivery. The transfer of the contours accounts for curving of the slices due to non-linearity of the slice selecting gradient field. The correction is done on the basis of an expansion into spherical harmonics of the slice selecting gradient field and can be done analytically. The invention may achieve correct transfer of contours defined in the 3D MRI data set during planning to contours employed for MR image guiding during RT delivery. Instead of the calculation of the true coordinates of the curved slice, the present subject matter may enable to complete the contour transfer by intentionally distorting the geometrically correct contours to match the curved coordinate system of the two-dimensional (2D) slices. This intentional distortion may require only the forward transformation, expressed analytically as spherical harmonic expansion of the slice selecting gradient field. After finding the distorted through-plane coordinate of each contour vertex, the remaining transform to the curved 2D slice can be found by interpolation. The invention can be implemented by an algorithm that is a part of the software associated with a product utilizing the invention.

The contour of an object refers to a shape of the object that segments object boundaries. The contour may be an outline of the object. The contour may be defined by vertices and edges connecting the vertices. The contour may be used to define a radiotherapy plan. If the contour transfer between the 3D MR image and 2D motion monitoring slice is performed taking into account the curvature of the 2D slice, the resulting contours may be accurate. This may especially be advantageous in the scenario where the radiation beam is gated based on the contours on the 2D slice, where inaccurate contours can lead to too high radiation dose to organs at risk or too low radiation dose to the target.

In one aspect, the invention relates to a medical analysis system for controlling a radiation therapy by a radiation therapy apparatus of a target volume in a subject. The medical analysis system comprises a memory for storing machine executable instructions, and a processor for controlling the medical analysis system, wherein execution of the machine executable instructions causes the processor to:
repeatedly monitor movement of the target volume during radiation of the target volume, the monitoring comprising:

receiving a two-dimensional, 2D, magnetic resonance, MR, image of the target volume for a slice selected according to a slice selecting gradient field, wherein the 2D MR image is geometrically corrected in the two in-plane dimensions of the 2D MR image;
determining a contour of the target volume in the 2D MR image, the determining of the contour comprising:

receiving a 3D contour drawn on a 3D MR image of the target volume, the 3D contour having first coordinates, wherein the 3D MR image is geometrically corrected in three dimensions of the 3D MR image;
distorting the first through plane coordinates of the 3D contour in accordance with a series expansion of the slice selecting gradient field, resulting in a distorted 3D contour having second coordinates;
determining in-plane coordinates of the distorted 3D contour on the selected slice by an interpolation of second coordinates;
using the determined contour and the 2D MR image for determining a possible movement of the target volume.

The target volume may, for example, be an organ such as a liver, kidney, prostate etc. The radiation of the target volume may be controlled on the basis of a radiotherapy plan. The radiotherapy plan may be defined on the basis of pre-recorded diagnostic 3D MR images. The present subject matter may update the radiotherapy plan based on the determined movement of the target volume. The radiation plan may be updated during the fractions of RT treatment e.g., to update the location of the target volume. For example, the radiation therapy apparatus may be Elekta Unity MR-Linac. Before radiation delivery, the acquisition of a daily 3D MR image may be performed. Next, while patient waits on the table, adaptations to organ contours and treatment plan may be performed based on the daily anatomy of the patient visualized by the 3D MR image. While radiation is being delivered, monitoring of organ motion based on individual dynamic 2D MR slices may be performed according to the present subject matter.

The 3D contour is drawn after the 3D MR image has been corrected. In particular, a geometric correction may be performed in the three dimensions of the 3D MR image. In addition, the received 2D MR image is corrected by application of a geometric correction in the two in-plane dimensions. Indeed, the 3D and 2D MR images may be distorted (curved) in all three dimensions. For that, the geometric corrections may be applied to the images after their reconstruction. For 3D MR images, the geometric correction may be performed in all three dimensions. For this reason, the contour drawn in the 3D image may be the true coordinates. Also, 2D MR images may geometrically be corrected in the two in-plane dimensions. However, the geometric correction in the third through-plane dimension may not be performed for 2D MR images, and thus the 2D MR images may always remain curved. This may be solved by the present subject matter without having to apply further corrections along the through-plane dimension, but instead to find out their distorted coordinates that are computationally easier to find. Indeed, as the slice curvature may be described by the spherical harmonic expansion of the slice selecting gradient, the calculation to find the true coordinates of the slice may require inversion of the spherical harmonic functions. This inverse may not be known analytically and its solution may require an iterative approach. Therefore, the inverse transformation may be computationally more laborious and also its implementation may be more complicated compared to the forward transformation as performed with the present subject matter. Furthermore, in case the 2D motion monitoring slice offsets are modified during the treatment delivery, e.g., to adapt to a drift of the monitored organ, the required contour transfer may need, without using the present subject matter, to be recalculated in real-time, placing strict restrictions on acceptable computational time. By contrast, the invention describes how the contour transfer can be calculated fast, without the need for the inverse transformation, using only the forward transformation.

The selected slice in each iteration may be a curved slice due to the non-linearity of the slice selection gradient. The first coordinates of the received 3D contour may be defined in a first coordinate system which is a true coordinate system, obtained by a state-of-the-art 3D geometric correction of the 3D image set before definition of the contour.

According to one embodiment, the selected slice is a curved slice due to a non-linearity of the slice selection gradient, wherein the selected slice has a through-plane center coordinate $z_0' = B_G(r_0, \varphi_0, \theta_0)/G$, where spherical coordinates $(r_0, \varphi_0, \theta_0)$ correspond to desired coordinates $(x_0, y_0, z_0)$ of the center of the slice, $G$ is the slice selection gradient strength at isocenter, $B_c$ is the slice selecting gradient field, the first coordinates of the 3D contour being $(x_i, y_i, z_i)$, wherein the execution of the machine executable instructions causes the processor to distort the z-coordinates of the received 3D contour as follows: $z_i' = B_G(r_i, \varphi_i, \theta_i)/G$, $(r_i, \varphi_i, \theta_i)$ being the spherical coordinates corresponding to the first coordinates $(x_i, y_1, z_i)$. In another embodiment, the selected slice coordinate $z_0'$ is changed one or more times during the radiation beam delivery. This may enable to better visualize anatomy that has moved out of the slice.

According to one embodiment, the received 3D contour comprises first vertices having the first coordinates $(x_i, y_i, z_i)$ and edges connecting the first vertices. The distorted 3D contour comprises second vertices having the second coordinates $(x_i, y_i, z_i')$ and edges connecting the second vertices, wherein the interpolation is performed along edges at the through-plane coordinate $z_0'$ in order to find the contour vertices $(x_j, y_j)$ on the selected curved slice.

According to one embodiment, the first coordinates are defined in a first coordinate system and the second coordinates are defined in a distorted coordinate system. The distorted coordinate system is provided such that the curved slice along the through plane in the first coordinate system becomes flat slice in the distorted coordinate system.

According to one embodiment, the execution of the machine executable instructions causes the processor to control the radiation therapy apparatus to adapt the radiation of the target volume based on the determined movement of the target volume. E.g., the radiotherapy plan may be updated based on the determined movement of the target volume for subsequent radiations using the updated radiotherapy plan.

According to one embodiment, the medical analysis system is remotely connected to the radiation therapy apparatus.

In one aspect, the invention relates to a magnetic resonance image-guided radiation therapy apparatus for controlling irradiation of a target volume. The apparatus comprises a magnetic resonance imaging system and the medical analysis system of any of the preceding embodiments. According to one embodiment, the execution of the machine executable instructions causes the processor to acquire 3D magnetic resonance data for the target volume using the magnetic resonance imaging system, reconstruct the 3D MR image, determine the 3D contour, and repeatedly acquire, during the radiation of the target volume, 2D magnetic resonance data for the target volume using the magnetic resonance imaging system, and reconstruct the 2D MR image.

In one aspect, the invention relates to a method for controlling a radiation therapy by a radiation therapy apparatus of a target volume in a subject. The method comprises:

repeatedly monitoring movement of the target volume during radiation of the target volume, the monitoring comprising:

receiving a two-dimensional, 2D, magnetic resonance, MR, image of the target volume for a slice selected according to a slice selecting gradient field, wherein the 2D MR image is geometrically corrected in the two in-plane dimensions of the 2D MR image;

determining a contour of the target volume in the 2D MR image, the determining of the contour comprising:

receiving a 3D contour drawn on a 3D MR image of the target volume, the 3D contour having first coordinates, wherein the 3D MR image is geometrically corrected in three dimensions of the 3D MR image;

distorting the first through plane coordinates of the 3D contour in accordance with a spherical harmonic expansion of the slice selecting gradient field, resulting in a distorted 3D contour having second coordinates;

determining in-plane coordinates of the distorted 3D contour on the selected slice by an interpolation of second coordinates;

using the determined contour and the 2D MR image for determining a possible movement of the target volume.

In another aspect, the invention relates to a computer program product comprising machine executable instructions for execution by a processor, wherein execution of the machine executable instructions causes the processor to the methods of any of the preceding embodiments.

It is understood that one or more of the aforementioned embodiments of the invention may be combined as long as the combined embodiments are not mutually exclusive.

## BRIEF DESCRIPTION OF THE DRAWINGS

In the following preferred embodiments of the invention will be described, by way of example only, and with reference to the drawings in which:

Fig. 1 is a schematic diagram of a medical analysis system in accordance with an example of the present subject matter,

Fig. 2 shows a cross-sectional view of a magnetic resonance image-guided radiation therapy apparatus in accordance with an example of the present subject matter,

Fig. 3 is a flowchart of a method for controlling a radiation therapy by a radiation therapy apparatus of a target volume in a subject,

Fig. 4 is a diagram illustrating the contour of the target volume in two coordinate systems.

## DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

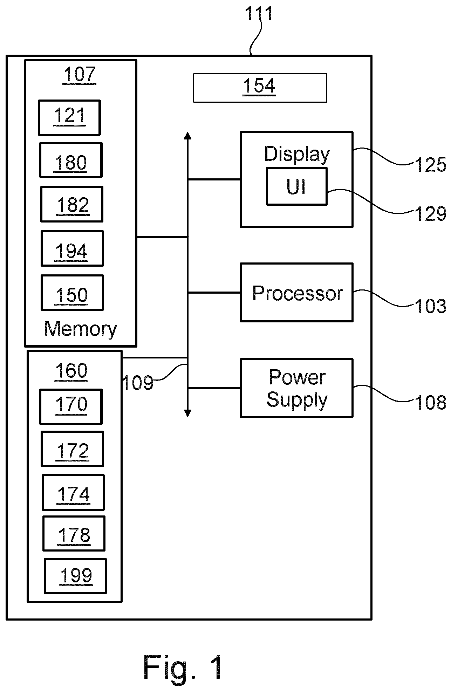

Fig. 1 is a schematic diagram of a medical analysis system 111 in accordance with an example of the present subject matter. The medical analysis system 111 comprises a processor 103, a memory 107 each capable of communicating with one or more components of the system 111. For example, components of the medical analysis system 111 are coupled to a bidirectional system bus 109.

It will be appreciated that the methods described herein may be at least partly non-interactive, and automated by way of computerized systems. For example, these methods can further be implemented in software 121, (including firmware), hardware, or a combination thereof. In exemplary embodiments, the methods described herein may be implemented in software, as an executable program, and executed by a special or general-purpose digital computer, such as a personal computer, workstation, minicomputer, or mainframe computer.

The processor 103 is a hardware device for executing software, particularly that stored in memory 107. The processor 103 can be any custom made or commercially available processor, a central processing unit (CPU), an auxiliary processor among several processors associated with the medical analysis system 111, a semiconductor based microprocessor (in the form of a microchip or chip set), a macroprocessor, or generally any device for executing software instructions. The processor 103 may control the operation of a magnetic resonance image-guided radiation therapy apparatus to which the medical analysis system 111 is connectable e.g. via a hardware interface 154.

The memory 107 can include any one or combination of volatile memory elements (e.g., random access memory (RAM, such as DRAM, SRAM, SDRAM, etc.)) and nonvolatile memory elements (e.g., ROM, erasable programmable read only memory (EPROM), electronically erasable programmable read only memory (EEPROM), programmable read only memory (PROM). Note that the memory 107 can have a distributed architecture, where various components are situated remote from one another, but can be accessed by the processor 103. Memory 107 may store an instruction or data related to at least one other constituent element of the system 111.

The medical analysis system 111 may further comprise a display device 125 which displays characters and images and the like e.g. on a user interface 129. The display device 125 may be a touch screen display device.

The medical analysis system 111 may further comprise a power supply 108 for powering the medical analysis system 111. The power supply 108 may for example be a battery or an external source of power, such as electricity supplied by a standard AC outlet.

The medical analysis system 111 may be configured to connect to a magnetic resonance image-guided radiation therapy apparatus (not shown) comprising a scanning imaging system such as MRI, CT and PET-CT imagers and a radiotherapy module.

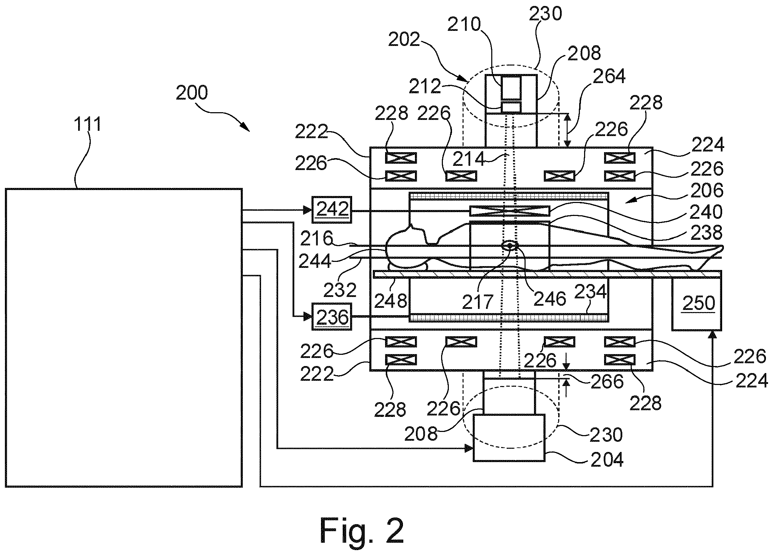

The connection between the medical analysis system 111 and the magnetic resonance image-guided radiation therapy apparatus may, for example, comprise a BUS Ethernet connection, WAN connection, Internet connection etc. The medical analysis system 111 and the magnetic resonance image-guided radiation therapy apparatus may or may not be an integral part. In other terms, the medical analysis system 111 may or may not be external to the magnetic resonance image-guided radiation therapy apparatus. The magnetic resonance image-guided radiation therapy apparatus comprises components that may be controlled by the processor 103 in order to configure the magnetic resonance image-guided radiation therapy apparatus. The configuration of the magnetic resonance image-guided radiation therapy apparatus may enable the operation of the magnetic resonance image-guided radiation therapy apparatus. The operation of the magnetic resonance image-guided radiation therapy apparatus may for example be automatic. Figure 2 shows an example of components of the magnetic resonance image-guided radiation therapy apparatus. In one example, the magnetic resonance image-guided radiation therapy apparatus may be configured to provide output data such as images in response to a specified measurement.

The processor 103 may be adapted to receive information from the magnetic resonance image-guided radiation therapy apparatus in a compatible digital form so that such information may be displayed on the display device 125. Such information may include operating parameters, alarm notifications, and other information related to the use, operation and function of the magnetic resonance image-guided radiation therapy apparatus.

Storage device 160 is shown as containing image magnetic resonance data 170 that have been acquired by the magnetic resonance imaging module. The storage device 160 is shown as further containing diagnostic images (i.e. image representation) 172 that have been reconstructed from the image magnetic resonance data. The storage device 160 is shown as further containing coordinates 174 of a target volume of a subject. The storage device 160 is shown as further containing radiotherapy control signals 178.

The memory 107 is shown as containing a therapy system control module 180. The therapy system control module 180 contains machine executable instructions which allow the processor 103 to control the overall functioning of the magnetic resonance image-guided radiation therapy apparatus. The memory 107 is shown as further containing a radiotherapy apparatus control module 182. The radiotherapy apparatus control module 182 contains machine executable instructions which allow the processor 103 to control the functioning of the radiotherapy module.

The memory 107 is shown as further containing radiotherapy control signal generation module 194. The radiotherapy control signal generation module 194 contains computer executable code which the processor 103 uses to generate the radiotherapy control signals 178. The radiotherapy control signals 178 may be generated in conjunction with the coordinates 174 of the target volume.

The memory 107 may further comprise a motion monitoring component 150 for performing at least part of the present method. The motion monitoring component 150 may or may not be part of software component 121. The motion monitoring component 150 may be configured to control at least part of the modules 180-194 for performing at least part of the present method.

The medical analysis system 111 may be configured to receive a treatment or irradiation plan 199 for irradiating the target volume, and may generate control signals e.g. using radiotherapy control signal generation module 194. The control signals may be used for controlling the radiotherapy module to irradiate the target volume using the control signals, in accordance with the location of the target volume.

Fig. 2 shows a cross-sectional view of an example magnetic resonance image-guided radiation therapy apparatus 200. The apparatus 200 is shown as comprising a radiotherapy module 202 and a magnetic resonance imaging module 206. The radiotherapy module 202 comprises a ring mechanism 208. The ring mechanism 208 supports a radiotherapy source 210. The radiotherapy source 210 is representative and may be a LINAC x-ray source, an x-ray 2 and a radioisotope gamma radiation source. Adjacent to the radiotherapy source 210 is a multi-leaf beam collimator 212 for collimating a radiation beam 214 that is generated by the radiotherapy source 210. The ring mechanism 208 is also adapted for moving e.g. rotating the radiotherapy source 210 and the beam collimator 212 about a rotational point 217 of the radiotherapy module 202. A rotational axis 216 passes through the rotational point 217.

The magnetic resonance imaging module 206 is shown as comprising a main magnet 222. The ring mechanism 208 is ring-shaped and surrounds the main magnet 222. The main magnet 222 shown in Figure 2 is a cylindrical type superconducting magnet. However, other magnets are also applicable for embodiments of the invention. The main magnet 122 has a supercooled cryostat 224. Inside the cryostat 224 there is a collection of superconducting coils 226. There are also compensation coils 228 whose current opposes the direction of current in the superconducting coils 226. This creates a low magnetic field zone 230 that circles or encompasses the main magnet 222. The cylindrical main magnet 222 is shown as having an axis 232 of symmetry.

Within the bore of the magnet there is a magnetic field gradient coil 234 which is used for acquisition of image magnetic resonance data to spatially encode objects within an imaging volume 238 of the main magnet 222. The magnetic field gradient coil 234 is connected to a magnetic field gradient coil power supply 236. The magnetic field gradient coil 234 is intended to be representative. Typically magnetic field gradient coils contain three separate sets of coils for spatially encoding in three orthogonal spatial directions. The imaging volume 238 is located in the center of the main magnet 222.

Adjacent to the imaging volume 238 is a radio frequency (RF) coil 240 for manipulating the orientations of magnetic spins within the imaging volume 238 and for receiving radio transmissions from spins also within the imaging volume 238. The radio frequency coil 240 is connected to a radio frequency transceiver 242. The radio frequency coil 240 and radio frequency transceiver 242 may be replaced by separate transmit and receive coils and a separate transmitter and receiver. It is understood that the radio frequency coil 240 and the radio frequency transceiver 242 are simply representative.

Within the center of the main magnet 222 is also located a subject 244. The subject 244 has a target volume (or target zone) 246 and is shown as reposing on a patient carrier 248. The RF coil 240 may transmit RF pulses into the target volume 246. The patient carrier 248 has a mechanical positioning system 250. The mechanical positioning system 250 is adapted for positioning the patient carrier 248 within the main magnet 222. Depending upon the space available inside of the main magnet 222, the mechanical positioning system 250 may move the patient carrier 248 in different directions including a direction perpendicular to the magnet axis 232. If there is more space available inside the main magnet 222 the mechanical positioning system 250 may have more degrees of freedom. For instance the mechanical positioning system 250 may position the patient carrier 248 with six degrees of freedom.

The radio frequency transceiver 242, the magnetic field gradient coil power supply 236, the mechanical actuator 204, and the mechanical positioning system 250 are all shown as being connected to the medical analysis system 111 e.g., via hardware interface 154.

The radiotherapy module 202 may be, for example, connected to the hardware interface 154 and communicates with the medical analysis system 111 via the mechanical actuator 204 or via other means enabling communication between radiotherapy module 202 and the medical analysis system 111.

For the example shown in Fig. 2, the rotational axis 216 of the radiotherapy module is not coaxial with the magnet axis 232. The rotational point 217 is shown as being off center from the magnet axis 232. It can be seen that the target zone 246 is off-center and away from the magnet axis 232. The radiotherapy apparatus 202 has been moved by mechanical actuator 204 such that the rotational point 217 of the radiotherapy module is within the target zone 246. It can be seen that the ring mechanism 208 has been moved relative to the magnet 222.

The radiation beam 214 passes through the rotational point 217. Placing the rotational point 217 at the center of the target zone 246 allows the target zone to be treated continuously when the radiation beam 214 is created by the radiotherapy source 210 and is rotated by the ring mechanism 208.

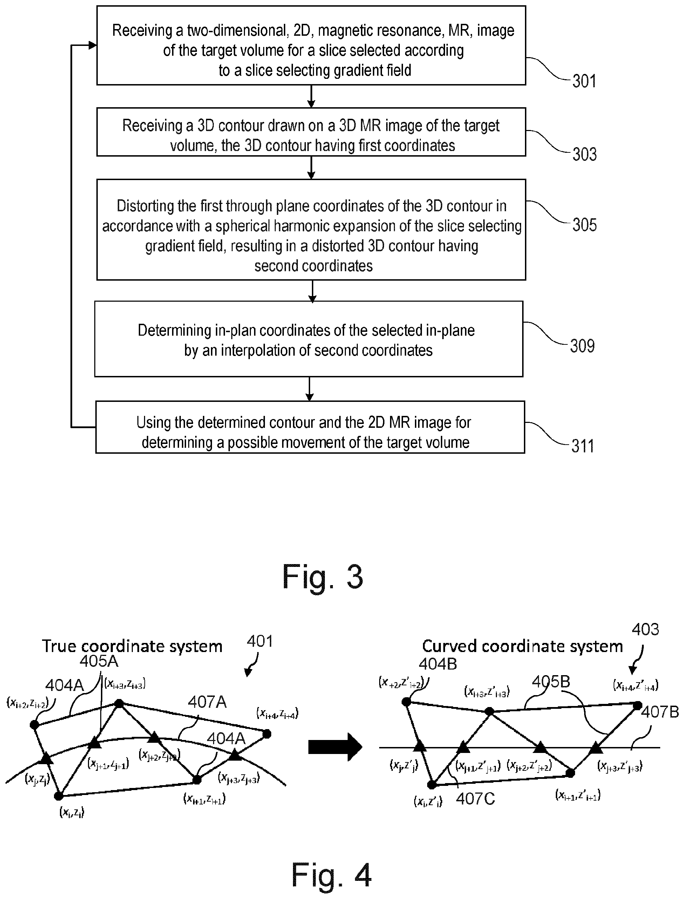

Fig. 3 is a flowchart of a method for controlling a radiation therapy e.g., by magnetic resonance image-guided radiation therapy apparatus 200, of the target volume 246.

A 2D MR image of the target volume 246 may be received in step 301. For example, the medical analysis system 111 may receive 2D MR data acquired by the magnetic resonance imaging module 206. The medical analysis system 111 may reconstruct the 2D MR image from the received 2D MR data and perform a known 2D geometric correction (e.g., such as the correction techniques cited in the background section) in the two in-plane dimensions of the 2D MR image. In another example, the medical analysis system 111 may receive the reconstructed 2D MR image from the magnetic resonance imaging module 206 and perform a 2D geometric correction in the two in-plane dimensions of the 2D MR image.

The 2D MR data may be acquired by the magnetic resonance imaging module 206 at a given slice (named a motion monitoring 2D slice) with the desired coordinates of its center at $(x_0, y_0, z_0)$ corresponding to spherical coordinates $(r_0, \varphi_0, \theta_0)$ using a slice selection gradient strength of G. The slice selecting gradient field may be defined as follows:

$$B_G(r, \varphi, \theta) = \sum_{l,m} \left(\frac{r}{r_{\text{ref}}}\right)^l P_{l,m}(\cos\theta) \left(C_{l,m}\cos\varphi + S_{l,m}\sin\varphi\right),$$

where $r$, $\varphi$, and $\theta$ are the spherical coordinates, $r_{ref}$ is the reference radius, and $P_{1,m}$ is the associated Legendre polynomial, $C_{1,m}$ and $S_{1,m}$ are the spherical harmonic coefficients describing the gradient field.

However, due to the non-linearity of the slice selection gradient, the motion monitoring 2D slice may be a curved slice because the effective z'-coordinates of the slice obtained by the slice selection gradient are not exactly the desired z-coordinates. The z'-coordinate may be defined as follows: $z'_j = B_G(r_k, \varphi_k, \theta_k)/G$ in accordance with a spherical harmonic expansion of the slice selecting gradient field. That is, the center of the motion monitoring 2D slice is selected at a coordinate $z_0' = B_G(r_0, \varphi_0, \theta_0)/G$ in a distorted coordinate system.

A 3D contour that has been drawn on a geometrically correct 3D MR image of the target volume 246 may be received in step 303. The 3D contour comprises vertices having coordinates $(x_1, y_i, z_i)$ in a true coordinate system, where i is an enumeration of the vertices e.g., as shown with the graph 401 of Figure 4. The coordinates $(x_i, y_i, z_i)$ may be coordinates of geometrically correct contour vertices. For example, a known geometric correction (e.g., such as the correction techniques cited in the background section) may be performed in the three dissensions of the 3D MR image.

The set of distorted z-coordinates of these vertices may be calculated in step 305 by $z'_i = B_G(r_i, \varphi_i, \theta_i)/G$, where $(r_i, \varphi_i, \theta_i)$ are spherical coordinates corresponding to coordinates $(x_i, y_i, z_i)$ of vertex i. This may result in a distorted 3D contour having second coordinates as shown in the graph 403 of Figure 4. The second coordinates of each vertex of the distorted 3D contour may be $(x_i, y_i, z'_i)$. The distortion of the 3D contour is performed in accordance with the spherical harmonic expansion of the slice selecting gradient field.

For example, the first coordinates (x, y, z) of the 3D contour may be the geometrically correct coordinates and may be expressed in a coordinate system named true coordinate system. A distorted coordinate system may be defined based on the true coordinate system by transforming all true z-coordinates defined in the true coordinate system by the relation $z' = B_G(r, \varphi, \theta)/G$, where $(r, \varphi, \theta)$ are spherical coordinates corresponding to the true or desired coordinates (x, y, z), wherein the coordinates that belong to a same curved plane in the true coordinating system would belong to a same flat plane in the distorted coordinate system.

In-plane coordinates of the distorted 3D contour on the motion monitoring 2D slice may be determined in step 309 by an interpolation of selected second coordinates. These in-plane coordinates of the motion monitoring 2D slice may be provided as coordinates of vertices of the contour of the target volume in the 2D MR image. The selected second coordinates may be second coordinates of vertices of selected contour edges (of the distorted 3D contour) that cross the motion monitoring 2D slice at the coordinate $z_0'$. In one example, the selected edges at the coordinate $z_0'$ may be all edges that cross the motion monitoring 2D slice at the coordinate $z_0'$. In one example, the selected edges at the coordinate $z_0'$ may be a subset of edges that cross the motion monitoring 2D slice at the coordinate $z_0'$ e.g., the edges may be selected randomly or by prompting a user to select them.

For example, these in-plane coordinates $(x_j, y_j)$ of the organ vertices on the curved slice may be found by a linear interpolation along a suitable contour edge at the coordinate $z_0'$. The suitable contour edge may not belong to the motion monitoring 2D slice, because if the suitable edge belongs to the motion monitoring 2D slice, then also the vertices it connects must belong to the motion monitoring 2D slice and therefore no interpolation may be needed. The determined in-plane coordinates may define vertices of the contour of the 2D MR image. Following the illustrated example of Figure 4, the suitable edge at the coordinate $z_0'$ may be the edge referenced by reference numeral 407C. An interpolation may be applied on the second coordinates $(x_{i+3}, y_{i+3})$ and $(x_i, y_i)$ of the vertices belonging to the edge 407C in order to find the in-plane coordinates.

As an example of the linear interpolation to find the coordinates of vertex $(x_{j+1}, y_{j+1}, z_{j+1}')$ on the slice in the distorted coordinate system, consider first the equation for edge 407C connecting vertices $(x_{i+3}, y_{i+3}, z_{i+3}')$ and $(x_i, y_i, z_i')$

$$x = x_i + k(x_{i+3} - x_i)$$

$$y = y_i + k(y_{i+3} - y_i)$$

$$z' = z_i + k(z_{i+3}' - z_i'),$$

where $0 \le k \le 1$ is a parametrization of the edge. It is easy to see by substitution that $k = 0$ corresponds to the vertex $(x_i, y_i, z_i')$ and $k = 1$ corresponds to $(x_{i+3}, y_{i+3}, z_{i+3}')$. These equations can be used to find the point $(x_{j+1}, y_{j+1}, z'_{j+1})$ belonging to the edge by first finding out the corresponding k by substituting $z_{j+1}'$ to the above equation for z, resulting in

$$k = \frac{z_{j+1}' - z_i'}{z_{i+3}' - z_i'}.$$

This k corresponding to vertex $(x_{j+1}, y_{j+1}, z_{j+1}')$ can be subsequently substituted in the equations for x and y as

$$x_{j+1} = x_i + \frac{z_{j+1}' - z_i'}{z_{i+3}' - z_i'}(x_{i+3} - x_i)$$

$$y_{j+1} = y_i + \frac{z_{j+1}' - z_i'}{z_{i+3}' - z_i'}(y_{i+3} - y_i).$$

From these equations, $(x_{j+1}, y_{j+1})$ can be calculated directly from the known coordinates of vertices $(x_{i+3}, y_{i+3}, z_{i+3}')$ and $(x_i, y_i, z_i')$ and the known coordinate $z_{j+1}' = z_0'$ of the slice in the curved coordinate system.

The same calculation may be repeated for all or a subset of edges crossing the curved slice.

The determined contour of the 2D MR image and the 2D MR image may be used in step 311 to determine whether the target volume has moved e.g., with respect to a last iteration of the method. The method steps 301 to 311 may be repeated multiple times during the radiation of the target volume, wherein in each iteration the 3D MR image may or may not be the same as the 3D MR image of the previous iteration. In case of a detected movement in a given iteration, the radiation of the target volume may be adapted accordingly for the subsequent iterations. The repetition of the method steps 301 to 311 may, for example, be performed until a predefined number of repetitions is reached. The coordinate $z_0'$ of the selected motion monitoring 2D slice may be the same in all iterations of the method steps 301 to 311. In another example, the coordinate $z_0'$ of the selected motion monitoring 2D slice may be changed for one or more iterations. This may enable to better visualize anatomy that has moved out of the slice.

In all of the above description, for simplicity z-axis has been chosen to be orthogonal to the selected slice. In reality, the slice may be selected in any orientation, including oblique orientations not orthogonal to any of the principal axes.

Fig. 4 is a diagram illustrating the contour of the target volume in two coordinate systems. The 3D contour 401 (to be transferred) may comprise vertices 404A having coordinates $(x_i, y_i, z_i)$ and edges 405A connecting the vertices 404A. The 3D contour represents the true coordinates because the geometry correction is already applied on the corresponding 3D MR image. For simplification of the drawing, only few vertices and edges are referenced and the y coordinates is assumed a fixed constant for all the vertices and edges in the Figure 4 e.g., the coordinates of the vertex 404A are $(x_{i+2}, y_{i+2}, z_{i+2})$ however only the coordinates $(x_{i+2}, z_{i+2})$ along x and z are shown for simplification of the drawing. After distorting the z-coordinates of the 3D contour 401, the new 3D contour 403 may be obtained. The new 3D contour comprises new vertices 404B having coordinates $(x_i, y_i, z_i^r)$ and edges 405B linking the vertices 404B. The line 407A indicates a cross-section of the selected slice. The triangles correspond to the contour vertices on the curved slice, where the desired but unknown coordinate $(x_j, y_j)$ can be found by interpolation along the contour edge at $z_0^r$. Note that because the contour is on the curved slice, $z'_j = z'_{j+1} = z'_{j+2} = z'_{j+3} = z'_0$. For example, to be able to visualize the contour 401 to the user who on the computer screen sees the 2D motion monitoring slice, the problem may be to find out what the original contour looks like on the 2D slice. This may correspond to finding out the coordinates of the triangles. That is, the coordinates $(x_j, y_j)$ of the triangles shown in graph 401 are the unknown to be found out.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 103 | processor |
| 107 | memory |
| 108 | power supply |
| 109 | bus |
| 111 | control system |
| 121 | software |
| 125 | display |
| 129 | user interface |
| 150 | motion compensation module |
| 170 | image magnetic resonance data |
| 172 | diagnostic images |
| 174 | coordinates |
| 178 | radiotherapy control signals |
| 180 | therapy system control module |
| 182 | radiotherapy apparatus control module |
| 194 | radiotherapy control signal generation module |
| 199 | irradiation plan |
| 200 | MR guided therapy apparatus |
| 202 | radiotherapy module |
| 204 | mechanical actuator |
| 206 | magnetic resonance imaging module |
| 208 | ring mechanism |
| 210 | radio therapy source |
| 212 | multi-leaf beam collimator |
| 214 | radiation beam |
| 216 | rotational axis |
| 217 | rotational point |
| 222 | main magnet |
| 224 | cryostat |
| 226 | superconducting coil |
| 228 | compensation coil |
| 230 | low magnetic field zone |
| 232 | magnet axis |
| 234 | magnetic field gradient coil |
| 236 | magnetic field gradient coil power supply |
| 238 | imaging volume |
| 240 | radio frequency coil |
| 242 | radio frequency transceiver |
| 244 | subject |
| 246 | target volume |
| 248 | patient carrier |
| 250 | mechanical positioning system |
| 301-311 | method steps |
| 401 | graph |

| 403 | graph |
|---|---|
| 404A-B | vertices |
| 405A-B | edges |
| 407A-B | slice planes. |

## Claims

1. A medical analysis system for controlling a radiation therapy by a radiation therapy apparatus of a target volume in a subject, the medical analysis system comprising a memory for storing machine executable instructions; and a processor for controlling the medical analysis system, wherein execution of the machine executable instructions causes the processor to:
repeatedly monitor movement of the target volume during radiation of the target volume, the monitoring comprising:

   receiving a two-dimensional, 2D, magnetic resonance, MR, image of the target volume for a slice selected according to a slice selecting gradient field, wherein the 2D MR image is geometrically corrected in two in-plane dimensions of the 2D MR image;
   determining a contour of the target volume in the 2D MR image, the determining of the contour comprising:

      receiving a 3D contour drawn on a 3D MR image of the target volume, the 3D contour having first coordinates, wherein the 3D MR image is geometrically corrected in three dimensions of the 3D MR image;
      distorting first through plane coordinates of the 3D contour in accordance with a series expansion of the slice selecting gradient field, resulting in a distorted 3D contour having second coordinates;
      determining in-plane coordinates of the distorted 3D contour on the selected slice by an interpolation of second coordinates;
      using the determined contour and the 2D MR image for determining a possible movement of the target volume.

2. The system of claim 1, the selected slice being a curved slice due to a non-linearity of the slice selection gradient, wherein the selected slice has a through-plane center coordinate $z'_0$ — $D_G(r_0, \varphi_0, 0_0)/G$, where spherical coordinates $(r_0, \varphi_0, 0_0)$ correspond to desired coordinates $(x_0, y_0, z_0)$ of the center of the slice, $G$ is the slice selection gradient strength, $B_G$ is the slice selecting gradient field, the first coordinates of the 3D contour being $(\kappa_i, y_i, z_i)$, wherein the execution of the machine executable instructions causes the processor to distort the z-coordinates of the received 3D contour as follows: $z'_i = B_G(r_i, \varphi_i, \theta_i)/G$, $(r_i, \varphi_i, \theta_i)$ being the spherical coordinates corresponding to the first coordinates $(X_i, y_i, z_i)$.

3. The system of claim 1 or 2, the received 3D contour comprising first vertices having the first coordinates $(x_i, y_i z_i)$ and edges connecting the first vertices, the distorted 3D contour comprising second vertices having the second coordinates $(x_i, y_i, z)$ and edges connecting the second vertices, wherein the interpolation is performed along edges at the through-plane coordinate $z'_0$.

4. The system of any of the preceding claims, the selected slice being a curved slice due to a non-linearity of the slice selection gradient, the first coordinates being defined in a first coordinate system and the second coordinates being defined in a distorted coordinate system, the distorted coordinate system being provided such that the curved slice along the through plane in the first coordinate system becomes flat slice in the distorted coordinate system.

5. The system of any of the preceding claims, the execution of the machine executable instructions causes the processor: to control the radiation therapy apparatus to adapt the radiation of the target volume based on the determined movement of the target volume.

6. The system of any of the preceding claims, the series expansion being a spherical harmonic expansion.

7. The system of any of the preceding claims, wherein the slice is selected at a through-plane center coordinate $z'_0$ that is changed one or more times during the radiation beam delivery.

8. The system of any of the preceding claims, the medical analysis system being remotely connected to the radiation therapy apparatus.

9. A magnetic resonance image-guided radiation therapy apparatus for controlling irradiation of a target volume, the apparatus comprising a magnetic resonance imaging system and the medical analysis system of any of the preceding claims 1-7.

10. The apparatus of claim 9, wherein execution of the machine executable instructions causes the processor to

    acquire 3D magnetic resonance data for the target volume using the magnetic resonance imaging system; and reconstruct the 3D MR image;
    determine the 3D contour;
    repeatedly acquire, during the radiation of the target volume, 2D magnetic resonance data for the target volume using the magnetic resonance imaging system, and reconstruct the 2D MR image.

11. A method for controlling a radiation therapy by a radiation therapy apparatus of a target volume in a subject, the method comprising:
repeatedly monitoring movement of the target volume during radiation of the target volume, the monitoring comprising:

    receiving a two-dimensional, 2D, magnetic resonance, MR, image of the target volume for a slice selected according to a slice selecting gradient field, wherein the 2D MR image is geometrically corrected in two in-plane dimensions of the 2D MR image;
    determining a contour of the target volume in the 2D MR image, the determining of the contour comprising:

        receiving a 3D contour drawn on a 3D MR image of the target volume, the 3D contour having first coordinates, wherein the 3D MR image is geometrically corrected in three dimensions of the 3D MR image;
        distorting first through plane coordinates of the 3D contour in accordance with a spherical harmonic expansion of the slice selecting gradient field, resulting in a distorted 3D contour having second coordinates;
        determining in-plane coordinates of the distorted 3D contour on the selected slice by an interpolation of second coordinates;
        using the determined contour and the 2D MR image for determining a possible movement of the target volume.

12. A computer program product comprising machine executable instructions for execution by a processor, wherein execution of the machine executable instructions causes the processor to perform the method of claim 11.

Fig. 1

Fig. 2

Receiving a two-dimensional, 2D, magnetic resonance, MR, image of the target volume for a slice selected according to a slice selecting gradient field ⏤301

Receiving a 3D contour drawn on a 3D MR image of the target volume, the 3D contour having first coordinates ⏤303

Distorting the first through plane coordinates of the 3D contour in accordance with a spherical harmonic expansion of the slice selecting gradient field, resulting in a distorted 3D contour having second coordinates ⏤305

Determining in-plan coordinates of the selected in-plane by an interpolation of second coordinates ⏤309

Using the determined contour and the 2D MR image for determining a possible movement of the target volume ⏤311

# Fig. 3

True coordinate system 401
405A
404A $(x_{i+3}, z_{i+3})$
$(x_{i+2}, z_{i+2})$ 407A
$(x_{i+4}, z_{i+4})$
404A
$(x_{j+1}, z_{j+1})$ $(x_{j+2}, z_{j+2})$
$(x_j, z_j)$ $(x_{j+3}, z_{j+3})$
$(x_{i+1}, z_{i+1})$
$(x_i, z_i)$

Curved coordinate system 403
404B
$(x_{+2}, z'_{i+2})$ $(x_{i+3}, z'_{i+3})$ 405B $(x_{i+4}, z'_{i+4})$
407B
$(x_j, z'_j)$ $(x_{j+1}, z'_{j+1})$ $(x_{j+2}, z'_{j+2})$ $(x_{j+3}, z'_{j+3})$
$(x_i, z'_i)$ 407C $(x_{i+1}, z'_{i+1})$

# Fig. 4

Europäisches
Patentamt
European
Patent Office
Office européen
des brevets

**EUROPEAN SEARCH REPORT**

Application Number

EP 21 18 3368

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KEESMAN RICK ET AL: "Correcting geometric image distortions in slice-based 4D-MRI on the MR-linac", MEDICAL PHYSICS., vol. 46, no. 7, 7 June 2019 (2019-06-07), pages 3044-3054, XP055875487, US ISSN: 0094-2405, DOI: 10.1002/mp.13602 * the whole document * | 1-12 | INV. G01R33/48 G01R33/565 A61N5/10 |
| A | S. ROYS, K. READ, K. HOSSEINZADEH, R. GULLAPALLI: "Correction of probe induced distortion of image and spectroscopic information for accurate prostatebrachytherapy", PROCEEDINGS OF THE INTERNATIONAL SOCIETY FOR MAGNETIC RESONANCE IN MEDICINE, vol. 10, 2328, 18 May 2002 (2002-05-18), XP040587061, * the whole document * | 1-12 | |
| A | B PETERSCH: "Effects of geometric distortion in 0.2T MRI on radiotherapy treatment planning of prostate cancer", RADIOTHERAPY AND ONCOLOGY, vol. 71, no. 1, 1 April 2004 (2004-04-01), pages 55-64, XP055012432, ISSN: 0167-8140, DOI: 10.1016/j.radonc.2003.12.012 * Chapters 2.1, 2.2, 2.3, 2.4, 3.2, 3.3, 4.1, 4.2, 4.3; figures 2,3,7 * | 1-12 | TECHNICAL FIELDS SEARCHED (IPC) G01R A61N |

The present search report has been drawn up for all claims

| Place of search | Date of completion of the search | Examiner |
|---|---|---|
| Munich | 22 December 2021 | Faber-Jurk, Sonja |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ANDREW JANKE ; HUAWEI ZHAO ; GARY J. COWIN ; GRAHAM J. GALLOWAY ; DAVID M. DODDRELL.** *Magnetic Resonance in Medicine,* 2004, vol. 52, 115-122